# EUROPÄISCHE PATENTSCHRIFT

(11) **EP 1 089 975 B1**
(45) Veröffentlichungstag und Bekanntmachung des Hinweises auf die Patenterteilung: **28.04.2004**
(21) Anmeldenummer: 99927879.9
(22) Anmeldetag: 04.06.1999
(51) Int. Cl.: C07D 213/00

(54) **VERFAHREN ZUR HERSTELLUNG VON SUBSTITUIERTEN PYRIDINCARBONSÄUREN**
IMPROVED METHOD FOR PRODUCING SUBSTITUTED PYRIDINE-CARBOXYLIC ACIDS
PROCEDE AMELIORE DE PREPARATION D'ACIDES PYRIDINE-CARBOXYLIQUES SUBSTITUES

(30) Priorität: 22.06.1998 AT 106998
(43) Veröffentlichungstag der Anmeldung: 11.04.2001
(73) Patentinhaber: DSM Fine Chemicals Austria Nfg GmbH & Co KG, 4021 Linz (AT)
(72) Erfinder: STEINBAUER, Gerhard, A-4470 Enns (AT); ZIMMERMANN, Curt, A-4310 Mauthausen (AT); WRESSNEGGER, Ernst, A-4030 Linz (AT); STEINWENDER, Erich, A-4040 Linz (AT)
(74) Vertreter: Lindinger, Ingrid
(86) Internationale Anmeldenummer: PCT/EP1999/003882
(87) Internationale Veröffentlichungsnummer: WO 1999/067217

(56) Entgegenhaltungen:
- C. O'MURCHU: SYNTHESIS,1989, Seiten 880-2, XP002117302 in der Anmeldung erwähnt

## Beschreibung

Substituierte Pyridincarbonsäuren sind wichtige Rohstoffe zur Synthese von Herbiziden, sodaß die Herstellung in technischem Maßstab von großer Bedeutung ist. Eine Methode zur Herstellung von substituierten Pyridincarbonsäuren ist die Ozonolyse der entsprechenden substituierten Chinoline (O'Murchu, Synthesis (1989) S. 880-882), wobei das Chinolin-Ausgangsprodukt, welches eine basische Funktion hat, durch Zusatz von Schwefelsäure als Sulfat in einem Gemisch aus Wasser und Essigsäure gelöst und die Ozonolyse in dieser Lösung durchgeführt wird. Je nach Substitution der Pyridincarbonsäure wird das Reaktionsgemisch mit Wasserstoffperoxid weiter oxidiert, insbesonders wenn substituierte Pyridin-2,3-dicarbonsäuren als Reaktionsprodukte erwünscht sind.

Ein technisch bedeutendes Produkt ist 2-Acetylnicotinsäure, welche durch Ozonolyse von 8-Methylchinolin hergestellt werden kann.
Bei der Durchführung einer entsprechenden Synthese in technischem Maßstab treten jedoch Nebenprodukte auf, die durch Kristallisation praktisch nicht aus dem Produkt entfernt werden können, womit die für die weitere Umsetzung geforderte hohe Reinheit nicht erreicht werden kann. Als Struktur derartiger schwer entfernbarer Nebenprodukte wurden am Pyridinring alkylierte substituierte Pyridincarbonsäuren gefunden. Im Falle der Ozonolyse von 8-Methylchinolin zur Herstellung von 2-Acetylnicotinsäure werden 2-Acetyl-4-methylnicotinsäure und 2-Acetyl-6-methylnicotinsäure gefunden, wobei das 4-Methyl-Derivat bei einer Kristallisation aus einem Lösungsmittel wie Ethylacetat, Methyl-tert.-butylether, Aceton, Tetrahydrofuran, Toluol, Methylisobutylketon, Butanol oder Wasser im Produkt verbleibt.

Die Ursache dieser Nebenproduktbildung wurde in einem Gehalt von wenigen ppm an Eisen im Reaktionsgemisch gefunden. In einer technischen Anlage, die zumindest teilweise aus Bestandteilen aufgebaut ist, deren Werkstoff Edelstahl ist, sind Spuren von Eisen praktisch nicht vemeidbar, insbesonders wenn, wie im vorliegenden Fall in wäßriger stark saurer Lösung gearbeitet wird.

Aufgabe der vorliegenden Erfindung war es daher ein verbessertes technisches Verfahren zur Herstellung von substituierten Pyridincarbonsäuren durch Ozonolyse eines entsprechend substituierten Chinolins zu finden, bei welchem die beschriebene Nebenproduktbildung auch in Gegenwart von Spuren an Metallen wie Eisen verhindert werden kann.

Gegenstand der Erfindung ist demnach ein verbessertes Verfahren zur Herstellung von substituierten Pyridincarbonsäuren durch Ozonolyse von Chinolinen, das dadurch gekennzeichnet ist, daß ein Chinolin der Formel 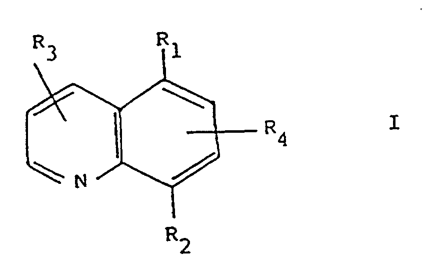 das in Position 2 und/oder 3 und/oder 4 durch R₃, sowie in Position 6 und/oder 7 durch R₄ substituiert ist, wobei R₁ und R₂ H oder eine C₁ - C₃ Alkylgruppe und R₃ und R₄ eine unter den Reaktionsbedingungen inerte Gruppe bedeuten und mindestens einer der Reste R₁ und R₂ kein H ist, in wäßriger saurer Lösung bei Temperaturen von -5 bis + 40°C mit Ozon umgesetzt, die so erhaltene Lösung zur Zersetzung der gebildeten Peroxide unter Einleitung von Sauerstoff oder Luft 0,5 bis 15 Stunden auf einer Temperatur von 0 bis 100 °C gehalten wird und die entsprechende substituierte Pyridincarbonsäure der Formel 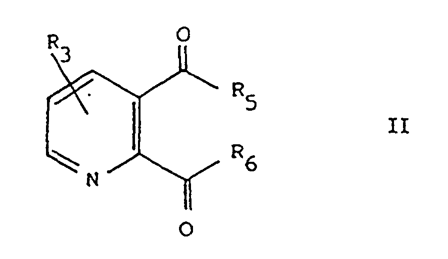 in der R₃ wie oben definiert ist und R₅ und R₆ OH oder C₁ bis C₃ Alkyl bedeuten, wobei mindestens einer der Reste R₅ und R₆ kein OH ist, aus dem Reaktionsgemisch isoliert wird.

Bei dem erfindungsgemäßen Verfahren werden Chinoline der Formel I zu substituierter Pyridincarbonsäuren der Formel II umgesetzt. Geeignete Chinoline sind dabei in Position 5 oder 8 mit einer Methyl-, Ethyl-, iso- oder n-Propylgruppe substituiert. Weiters können die Chinoline in Position 2 und/oder 3 und/oder 4 durch Wasserstoff, C₁ - C₃-Alkyl- oder Alkoxygruppen, Halogen u.s.w. substituiert sein. Bevorzugt ist nur eine der Positionen 2, 3 oder 4 substituiert, besonders bevorzugt weisen die als Edukt verwendeten Chinoline in Position 2, 3 und 4 Wasserstoff als Substituenten auf. Die als Edukte geeigneten Chinoline können auch in Position 6 und/oder 7 durch eine unter den Reaktionsbedingungen inerte Gruppe, wie etwa durch eine C₁ - C₃-Alkyl- oder Alkoxygruppe, Halogen, u.s.w., substituiert sein. Weiters sind solche Chinoline bevorzugt, die in Position 8 durch eine Methyl- oder Ethylgruppe substituiert sind und wobei die Position 5 durch Wasserstoff besetzt ist. Beispiele dafür sind 8-Methylchinolin, 3-Ethyl-8-methylchinolin. Besonders bevorzugt wird 8-Methylchinolin verwendet.

Die Ausgangsprodukte sind dabei entweder käuflich erhältlich oder können z.B. durch Skraup-Synthese, wie etwa in C.O'Murchu, Synthesis 1989, S. 880 - 882 beschrieben, hergestellt werden.

Die entsprechende Reaktion wird erfindungsgemäß in wäßriger saurer Lösung durchgeführt. Als Säure eignen sich dabei z.B. Mineralsäuren wie Schwefelsäure, Salpetersäure oder Phosphorsäure.
Gegebenenfalls kann noch ein zusätzliches Lösungsmittel wie etwa Essigsäure, Methanol, u.s.w. zugesetzt werden. Auf die Verwendung von Essigsäure wird jedoch bevorzugt verzichtet, sodaß die Ozonolyse ausschließlich in wäßriger Mineralsäure als Lösungsmittel für das substituierte Chinolin durchgeführt wird. Besonders bevorzugt wird eine wäßrige Schwefelsäurelösung eingesetzt. Die Menge an Mineralsäure ist von geringer Bedeutung. Wird außer der wäßrigen Mineralsäure kein weiteres Lösungsmittel (wie z.B. Essigsäure) verwendet, muß naturgemäß eine ausreichende Menge an Mineralsäure zur Bildung eines Salzes des Chinolins verwendet werden, das sind im Fall von Schwefelsäure 0,5 Äquivalente oder im Fall von Salpetersäure 1 Äquivalent bezogen auf das substituierte Chinolin, um so eine homogene Lösung des Ausgangsgemisches zu erreichen.
Das Ausgangsprodukt wird in der wäßrigen sauren Lösung gelöst, wobei die Konzentration an Edukt zwischen 2 und 30 Gew.%, bevorzugt zwischen 2,5 und 10 Gew.% liegen sollte. Niedrigere Konzentrationen an Edukt erhöhen die Ausbeute an dem gewünschten Endprodukt. Die so erhaltene Lösung wird solange mit einem ozonführenden O₂-Strom begast bis die äquivalente Menge an Ozon bzw. ein Überschuß aufgenommen wurde. Das Ende und somit die Dauer der Reaktion ist durch den Verbrauch der theoretischen Ozonmenge gegeben und läßt sich weiters durch einen gleichzeitig auftretenden erhöhten Ozondurchbruch leicht feststellen.
Das Ende der Reaktion kann femer leicht durch eine geeignete Inprozeßkontrolle auf das Abreagieren des substituierten Chinolins festgestellt werden.

Die Temperatur der Ozonolyse beträgt -5 bis +40 °C. Bevorzugt wird eine Temperatur von 0 bis +10 °C gewählt. Anschließend an die Ozonolyse werden die intermediär entstehenden Peroxide durch Erwärmen der Lösung zersetzt, wobei die gewünschte substituierte Pyridincarbonsäure entsteht. Die Temperatur bei der Peroxidzersetzung kann zwischen 0 und 100 °C liegen, bevorzugt bei etwa 50 bis 70 °C. Naturgemäß hängt die Dauer der Peroxidzersetzung von der gewählten Temperatur ab und dauert beispielsweise bei 60 °C ca. 2,5 Stunden. Bei dem erfindungsgemäßen Verfahren ist zur Peroxidzersetzung kein weiteres Oxidationsmittel wie Wasserstoffperoxid notwendig.
Gleichzeitig wird Sauerstoff während der Dauer der Peroxidzersetzung in die Reaktionslösung eingeleitet. Sauerstoff kann dabei in Form von reinem Sauerstoff oder in Form von Luft verwendet werden. Durch diese Maßnahme wird die Bildung von in Position 4- oder 6-alkylierten Endprodukte verhindert. Die Peroxidzersetzung wird bis zum Erreichen eines Peroxidrestgehaltes von maximal 5 mmol/l durchgeführt. Ein Rest an Peroxiden kann auch durch Zugabe eines Reduktionsmittels wie z.B. Natriumpyrosulfit vor der weiteren Aufarbeitung zerstört werden. Befinden sich nur sehr geringe ppm-Mengen an Eisen im Reaktionsgemisch so kann die Bildung der in 4- bzw. 6-Stellung durch Alkylgruppen substituierte Pyridincarbonsäuren durch Abbrechen der Peroxidzersetzung und Reduktion vorhandener Peroxidreste durch Reduktionsmittel minimiert werden, da die radikalische Nebenreaktion bevorzugt am Ende der Peroxidzersetzung stattfindet.
In diesem Fall kann gegebenenfalls auf die Sauerstoffeinleitung verzichtet werden. Der Nebenproduktgehalt sollte 0,1 Gewichtsprozent (bestimmt z.B. mittels HPLC oder GC) nicht übersteigen.
Das gewünschte Endprodukt wird mittels Extraktion aus der Reaktionslösung isoliert. Der pH-Wert während der Extraktion sollte dabei unter 4, bevorzugt unter 2,5 liegen. Die Einstellung des gewünschten pH-Wertes erfolgt bevorzugt mittels Natrium- oder Kaliumhydroxid.

Als Lösungsmittel zur Extraktion eignen sich bevorzugt Toluol, Methyl-tert.butylether, Ethylacetat oder n-Butanol.
Besonders bevorzugt wird Ethylacetat oder Methyl-tert.-butylether als Extraktionsmittel eingesetzt. Anschließend an die Extraktion wird die organische Phase eingeengt, bevorzugt auf eine Konzentration von 10 bis 30 Gew.% an Produkt, und bei -10 bis +10°C das gewünschte Endprodukt auskristallisiert.
Während des Abdampfens des organischen Lösungsmittels erfolgt für den Fall, daß dieses Lösungsmittel mit Wasser ein Azeotrop bildet, auch das azeotrope Entfernen von Wasser.

Durch das erfindungsgemäße Verfahren werden die gewünschten substituierten Pyridincarbonsäuren der Formel II in Ausbeuten von 70 - 80 % erhalten. Die Reinheit der Produkte liegt bei >98 %. Das erfindungsgemäße Verfahren wird bevorzugt zur Herstellung von 2-Acetylnicotinsäure (ANA) verwendet.
ANA wird dabei in Ausbeuten von 70 - 75 % und einer Reinheit von >98 % erhalten. Störende Nebenprodukte wie 2-Acetyl-4-methylnicotinsäure treten nicht bzw. nur in vernachlässigbaren Mengen auf.
Die erfindungsgemäß hergestellten Pyridincarbonsäuren eigenen sich aufgrund ihrer Reinheit besonders gut als Ausgangsprodukt zur Herstellung von Herbiziden, bevorzugt eignet sich 2-Acetylnicotinsäure zur Herstellung von Herbiziden auf Basis von substituierten Semicarbazonen.

### Beispiel 1 (Vergleichsversuch)

12 kg 8-Methylchinolin (84 mol) wurden in 250 Liter Wasser und 9,5 kg 60 %iger Salpetersäure (90 mol) gelöst. Die Lösung wurde auf 1 °C abgekühlt und in diese Lösung ein Sauerstoffstrom, welcher 60 g/m³ Ozon enthielt, eingeleitet. Dies erfolgte so lange, bis der Restgehalt an 8-Methylchinolin in der Lösung ca. 1 g/l betrug (Bestimmung mittels GC). Anschließend wurde die Lösung zur Zersetzung der Peroxide 4 Stunden auf 60 °C erwärmt. Der Endpunkt der Peroxidzersetzung wurde mittels Titration (Kaliumjodid, Natriumthiosulfat, Stärke) bestimmt. Der Peroxidrestgehalt betrug 1 mmol/l.
Drei identisch durchgeführte Chargen wurden vereinigt. In der Peroxidlösung wurde ein Gehalt von 12 ppm Eisen gefunden.
Es wurde mit 50 %iger Natronlauge ein pH von 1 eingestellt und die Lösung über einen Siebbodenextraktor im Gegenstrom im Phasenverhältnis 1/1 mit Ethylacetat extrahiert. Das Extrakt wurde durch Abdestillieren von Ethylacetat auf ein Volumen von ca. 180 Liter eingeengt. Die Lösung wurde auf -5 °C abgekühlt, das Produkt über eine Drucknutsche abfiltriert, mit vorgekühltem Ethylacetat gewaschen und auf der Drucknutsche im Vakuum getrocknet.
Es wurden 28 kg (67 % d. Th.) 2-Acetylnicotinsäure erhalten, welches laut GC und HPLC 0,75 % 2-Acetyl-4-methylnicotinsäure enthielt.

Auf diese Weise wurden Chargen mit bis zu 9,1 % 2-Acetyl-4-methylnicotinsäure erhalten.

### Beispiel 2:

In 300 ml einer analog Beispiel 1 hergestellten ozonisierten Lösung wurde ein Sauerstoffstrom eingeleitet. Es wurden 40 mg Eisen(II)sulfat Heptahydrat, entsprechend einem Gehalt von 25 ppm Eisen in der Lösung zugegeben und das Gemisch 2,5 Stunden auf 60 °C unter weiterem Einleiten von Sauerstoff erwärmt. Nach Titration wurde ein Peroxidgehalt von 3 mmol/l gefunden. Nach pH-Stellen auf pH = 1 mit 50 %iger Natronlauge, Extraktion mit Ehtylacetat wurde 2-Acetylnicotinsäure erhalten, in welchem kein Nebenprodukt 2-Acetyl-4-methylnicotinsäure nachweisbar war.

### Beispiel 3:

250 kg 8-Methylchinolin (1,75 kmol) wurden in 3200 Liter Wasser und 180 kg 96 %ige Schwefelsäure (1,75 kmol) gelöst. Die Lösung wurde auf eine Temperatur von 1 °C abgekühlt und anschließend ein Sauerstoffstrom, welcher 50 bis 60 g/m³ Ozon enthielt, eingeleitet. Dies erfolgte solange, bis der Restgehalt an 8-Methylchinolin ca. 1 Gramm/Liter betrug (Bestimmung mittels GC)
Nach der Ozonolyse wurde das Reaktionsgemisch in einen Kessel, welcher 2000 Liter Wasser mit einer Temperatur von 60°C enthielt, abgelassen. In die wäßrige Lösung wurde während der Peroxidzersetzung ständig 2 m³ pro Stunde Luft eingeleitet. Die Peroxidzersetzung erfolgte 2 Stunden lang bei einer Temperatur von 60°C und erfolgte bis zu einem Peroxidrestgehalt von 3 bis 5 mmol/Liter (Titration).
Es wurde mit 50%iger Natronlauge ein pH-Wert von 1,5 bis 2 eingestellt und die Lösung über einen Siebbodenextraktor mit Methyl-tert.-butylether im Gegenstrom im Phasenverhältnis Methyl-tert.-butylether/wäßriger Lösung = 1,5/1 extrahiert. Das Extrakt wurde durch Abdestillieren von Methyl-tert.-butylether auf eine Konzentration von ca. 10 Gew.% eingeengt.
Die Lösung wurde auf -10°C gekühlt, das Produkt über eine Drucknutsche filtriert, mit vorgekühltem Methyl-tert-butylether gewaschen und auf der Nutsche im Vakuum getrocknet.

Es wurden 202 kg (70 % d. Th.) 2-Acetylnicotinsäure erhalten.
Die Reinheit laut HPLC betrug 98,5 %; es war keine 2-Acetyl-4-methylnicotinsäure als Nebenprodukt nachweisbar.

## Patentansprüche

1. Verbessertes Verfahren zur Herstellung von substituierten Pyridincarbonsäuren durch Ozonolyse von Chinolinen, **dadurch gekennzeichnet, dass** ein Chinolin der Formel 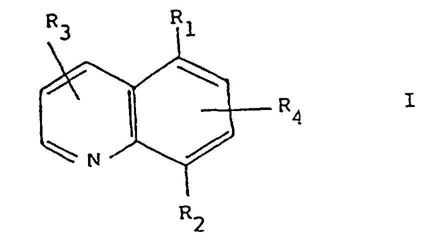 das in Position 2 und/oder 3 und/oder 4 durch R₃, sowie in Position 6 und/oder 7 durch R₄ substituiert ist, wobei R₁ und R₂ H oder eine C₁ - C₃ Alkylgruppe und R₃ und R₄ eine unter den Reaktionsbedingungen inerte Gruppe bedeuten und mindestens einer der Reste R₁ und R₂ kein H ist, in wäßriger saurer Lösung bei Temperaturen von -5 bis + 40°C mit Ozon umgesetzt, die so erhaltene Lösung zur Zersetzung der gebildeten Peroxide unter Einleitung von Sauerstoff oder Luft 0.5 bis 15 Stunden auf einer Temperatur von 0 bis 100 °C gehalten wird und die entsprechende substituierte Pyridincarbonsäure der Formel 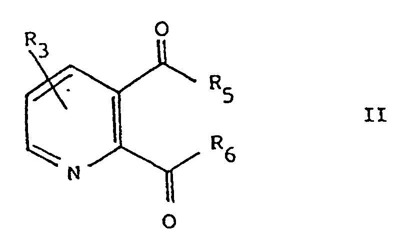 in der R₃ wie oben definiert ist und R₅ und R₆ OH oder C₁ bis C₃ Alkyl bedeuten, wobei mindestens einer der Reste R₅ und R₆ kein OH ist, aus dem Reaktionsgemisch isoliert wird.

2. Verfahren nach Anspruch 1, **dadurch gekennzeichnet, daß** als Chinolin der Formel I 8-Methylchinolin, 3-Ethyl-8-methylchinolin eingesetzt wird.

3. Verfahren nach Anspruch 1, **dadurch gekennzeichnet, daß** als wäßrige saure Lösung eine wäßrige Schwefel-, Salpeter- oder Phosphorsäurelösung eingesetzt wird.

4. Verfahren nach Anspruch 1, **dadurch gekennzeichnet, daß** die Umsetzung mit Ozon bei 0 bis +10 °C durchgeführt wird.

5. Verfahren nach Anspruch 1, **dadurch gekennzeichnet, daß** die Peroxidzersetzung bei 50 - 70 °C durchgeführt wird.

6. Verfahren nach Anspruch 1, **dadurch gekennzeichnet, daß** Sauerstoff in Form von reinem Sauerstoff oder in Form von Luft eingeleitet wird.

7. Verfahren nach Anspruch 1, **dadurch gekennzeichnet, daß** die substituierte Pyridincarbonsäure mittels Extraktion aus dem Reaktionsgemisch isoliert wird.

## Claims

1. Improved process for the preparation of substituted pyridinecarboxylic acids by ozonolysis of quinolines, **characterized in that** a quinoline of the formula 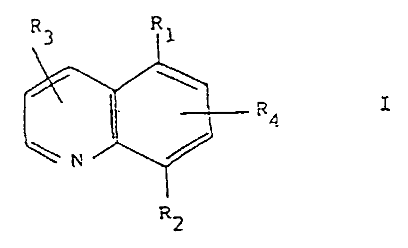 which is substituted in position 2 and/or 3 and/or 4 by R₃, and in position 6 and/or 7 by R₄, where R₁ and R₂ are H or a C₁-C₃-alkyl group and R₃ and R₄ are a group which is inert under the reaction conditions, and at least one of the radicals R₁ and R₂ is not H, is reacted with ozone in aqueous acidic solution at temperatures of from -5 to +40°C, the resulting solution is maintained at a temperature of from 0 to 100°C for 0.5 to 15 hours with the introduction of oxygen or air for decomposition of the peroxides formed, and the corresponding substituted pyridinecarboxylic acid of the formula 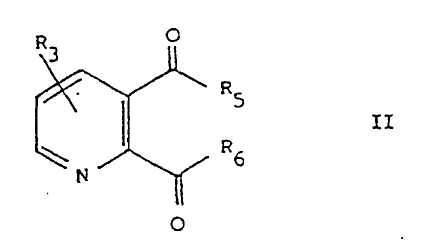 in which R₃ is as defined above, and R₅ and R₆ are OH or C₁ to C₃-alkyl, where at least one of the radicals R₅ and R₆ is not OH, is isolated from the reaction mixture.

2. Process according to Claim 1, **characterized in that** the quinoline of the formula I used is 8-methylquinoline or 3-ethyl-8-methylquinoline.

3. Process according to Claim 1, **characterized in that** the aqueous acidic solution used is an aqueous sulphuric acid, nitric acid or phosphoric acid solution.

4. Process according to Claim 1, **characterized in that** the reaction with ozone is carried out at 0 to +10°C.

5. Process according to Claim 1, **characterized in that** the peroxide decomposition is carried out at 50-70°C.

6. Process according to Claim 1, **characterized in that** oxygen is introduced in the form of pure oxygen or in the form of air.

7. Process according to Claim 1, **characterized in that** the substituted pyridinecarboxylic acid is isolated from the reaction mixture by means of extraction.

## Revendications

1. Procédé amélioré de préparation d'acides pyridine-carboxyliques substitués par ozonolyse de chinolines, **caractérisé en ce qu'**une chinoline de formule 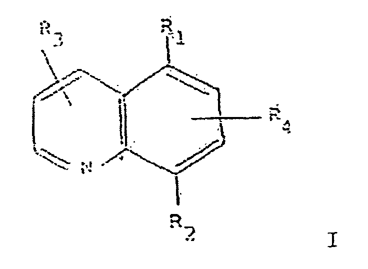 qui est substituée en position 2 et/ou 3 et/ou 4 par R₃, ainsi qu'en position 6 et/ou 7 par R₄, R₁ et R₂ signifiant l'hydrogène ou un groupe alkyle en C₁ à C₃ et R₃ et R₄ des groupes inertes dans les conditions de la réaction, et au moins l'un des radicaux R₁ et R₂ n'étant pas un hydrogène, est convertie en solution aqueuse acide à des températures de -5 à +40°C avec de l'ozone, que la solution ainsi obtenue pour la décomposition des peroxydes est maintenue sous introduction d'oxygène ou d'air 0,5 à 15 heures à une température de 0 à 100°C et que l'acide pyridine-carboxylique substitué correspondant de formule 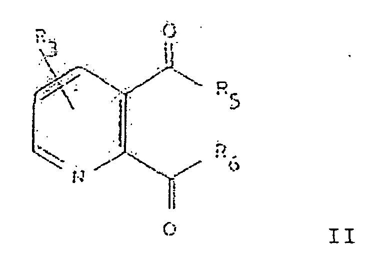 dans lequel R₃ est défini comme ci-dessus et R₅ et R₆ signifient OH ou un alkyle en C₁ et C₃, au moins, l'un des radicaux R₅ et R₆ n'étant pas un groupe OH, est isolé du mélange de réaction.

2. Procédé selon la revendication 1, **caractérisé en ce que** l'on a recours comme chinoline de formule I à la 8-méthylcholine ou à la 3-éthyl-8-méthylcholine.

3. Procédé selon la revendication 1, **caractérisé en ce que** l'on a recours comme solution aqueuse acide à une solution aqueuse d'acide sulfurique, nitrique ou phosphorique.

4. Procédé selon la revendication 1, **caractérisé en ce que** la conversion avec l'ozone est réalisée de 0 à +10°C.

5. Procédé selon la revendication 1, **caractérisé en ce que** la décomposition des peroxydes est réalisée de 50 à 70°C.

6. Procédé selon la revendication 1, **caractérisé en ce que** l'oxygène est introduit sous forme d'oxygène pur ou sous forme d'air.

7. Procédé selon la revendication 1, **caractérisé en ce que** l'acide pyridine-carboxylique substitué est isolé du mélange de réaction par extraction.
